# EUROPEAN PATENT SPECIFICATION

(11) **EP 4 062 426 B1**
(45) Date of publication and mention of the grant of the patent: **10.12.2025**
(21) Application number: 19809733.9
(22) Date of filing: 19.11.2019
(51) Int. Cl.: G16H 50/50

(54) **APPARATUS AND METHOD FOR LOCATING CELLS GENERATING AN ELECTRICAL ANOMALY IN A SUBJECT'S BODY PART**
VORRICHTUNG UND VERFAHREN ZUR LOKALISIERUNG VON ZELLEN, DIE EINE ELEKTRISCHE ANOMALIE IN EINEM KÖRPERTEIL EINES PATIENTEN ERZEUGEN
APPAREIL ET MÉTHODE DE LOCALISATION DE CELLULES GÉNÉRANT UNE ANOMALIE ÉLECTRIQUE DANS UNE PARTIE DU CORPS D'UN INDIVIDU

(43) Date of publication of application: 28.09.2022
(73) Proprietor: Ecole Polytechnique Fédérale de Lausanne (EPFL), 1015 Lausanne (CH)
(72) Inventor: SOROUCHYARI, Esfandiar, 1006 Lausanne (CH); RACHIDI-HAERI, Farhad, 1009 Pully (CH); DADRAS, Ali, 906 55 Umeå (SE)
(74) Representative: Vesterinen, Jussi Tapio
(86) International application number: PCT/EP2019/081744
(87) International publication number: WO 2021/098948

(56) References cited:
- US-A1- 2004 162 550
- SANJIV M. NARAYAN ET AL: "Clinical Mapping Approach To Diagnose Electrical Rotors and Focal Impulse Sources for Human Atrial Fibrillation", JOURNAL OF CARDIOVASCULAR ELECTROPHYSIOLOGY., vol. 23, no. 5, 26 April 2012 (2012-04-26), US, pages 447 - 454, XP055314832, ISSN: 1045-3873, DOI: 10.1111/j.1540-8167.2012.02332.x

## Description

### TECHNICAL FIELD

The present invention relates to an apparatus for locating source cells generating an electrical anomaly, such as atrial arrhythmia. More specifically, the apparatus is configured to use time-reversing of electrical signals when locating the source cells. The invention also relates to a corresponding system, method and computer program product for implementing the method.

### BACKGROUND OF THE INVENTION

Atrial arrhythmia, also known as atrial fibrillation, caused by malfunctioning of the sinoatrial node activity, is a very frequent source of heart disorders in humans. They can create long term damages that can end up in stroke or heart failure. One of the causes of atrial arrhythmia is the production of chaotic or irregular electrical impulses by heart cells other than the sinoatrial node (the natural pacemaker of the heart) that propagate chaotically through the atria, causing the distortion of heart beats.

In some types of treatment, doctors choose to cauterise and burn the cells responsible for chaotic electrical impulses by introducing a catheter and locating those cells. Radiofrequencies are usually used to burn those cells. The correct positioning and aiming of the catheter are therefore essential. Usually, mapping catheter is introduced in the heart which measures the electrical signal to locate cells to burn. The quality of recording of the electrical signal is therefore very important. However, it is currently difficult to record high quality signals and therefore the quality or precision of targeting faulty cells is low. Moreover, the process is an "exploration" of the heart tissue during which tachycardia may be induced and the results recorded. Further disadvantages of the known processes are invasive nature and length of these processes.

SANJIV M. NARAYAN ET AL. (JOURNAL OF CARDIOVASCULAR ELECTROPHYSIOLOGY, vol. 23, no. 5, 26 April 2012, pages 447-454, XP055314832) disclose a clinical mapping approach to diagnose electrical rotors and focal impulse sources for human atrial fibrillation. The disclosed approach does not involve time-reversing of one or more electrical signals and feeding the same into a simulation model.

### SUMMARY OF THE INVENTION

It is an object of the present invention to overcome at least some of the problems identified above related to detecting cells of a subject's body part, which generate abnormal electrical activity.

According to a first aspect of the invention, there is provided an apparatus for locating cells responsible for an electrical anomaly in a subject as recited in claim 1.

The present invention aims to find the location of faulty or malfunctioning cells in the subject's body part, such as the human heart in the case of arrhythmia for instance, by using time-reversal techniques in a computer or computer-based model of the body part. The proposed method aims to increase the precision of cell location detection and therefore also the precision of the optionally resulting intervention by providing a non-invasive method of detection. It can enhance the efficiency of the intervention and reduce the time of any subsequent intervention.

Presently, during heart interventions for instance, a catheter is used to record the heart activity and detect faulty cells to burn. The intervention must be short in time and therefore, the detection process can "miss" some "targets". It is known that atrial fibrillation can occur during a low, normal or high physical activity phase. During a traditional intervention, the heart is in its low activity phase. The present invention, when used for detecting abnormal heart cells, uses real heart activity signals. Therefore, these signals can be recorded and used before the surgical intervention in order to study and localise the source of faulty signals in advance. They can be recorded during low, normal or high activity phases outside the stress and emergency of any intervention. The recordings can subsequently be time-reversed according to the present invention in order to find all possible faulty cells during different activity phases of the heart.

According to a second aspect of the invention, there is provided a system for locating cells responsible for an electrical anomaly in a subject, the system comprising the data processing apparatus according to the first aspect, and a sensor system comprising a set of sensors for recording the one or more electrical signals resulting from electrical activity of the subject.

According to a third aspect of the invention, there is provided a method for locating source cells responsible for an electrical anomaly in a subject as recited in claim 14.

According to a fourth aspect of the invention, there is provided a computer program product comprising instructions for implementing the steps of the method according to the third aspect when loaded and run on computing means of a computing device.

Other aspects of the invention are recited in the dependent claims attached hereto.

### BRIEF DESCRIPTION OF THE DRAWINGS

Other features and advantages of the invention will become apparent from the following description of a non-limiting example embodiment, with reference to the appended drawings, in which:
- Figure 1 shows schematically some hardware and software components, which may be used to implement the cell detection method according to an example of the present invention;
- Figure 2 is a flow chart illustrating the cell detection method according to an example of the present invention; and
- Figure 3 is another flow chart schematically illustrating the cell detection method outlined in the flow chart of Figure 2.

### DETAILED DESCRIPTION OF AN EMBODIMENT OF THE INVENTION

An embodiment of the present invention will now be described in detail with reference to the attached figures. This embodiment is described in the context of detecting or locating human heart cells generating abnormal electrical signals, but the teachings of the invention are not limited to this environment. For instance, the teachings of the present invention could be used to detect one or more abnormal or faulty cells of another organ or body part that generate abnormal or faulty electrical signals. Identical or corresponding functional and structural elements which appear in the different drawings are assigned the same reference numerals.

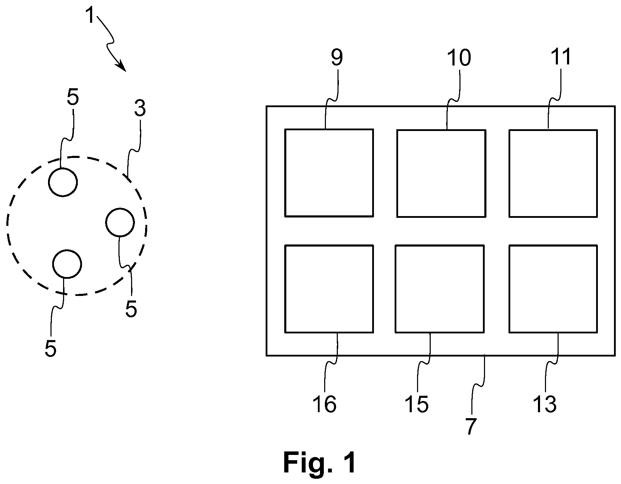

Figure 1 schematically illustrates an example cell detection or location system 1 for detecting abnormally functioning cells in a subject, which in this example is the human heart. The system in this example comprises two separate hardware units or modules, namely a sensor system 3 comprising one or more measurement sensors 5, and a data processing apparatus, device or system 7. The sensors 5 are connected to the data processing apparatus either by using a wireless connection or a wired connection. Thus, the sensor system or more specifically the individual sensors may comprise a communication interface module or unit for this purpose, which is however not illustrated in Figure 1. The sensors, which in this example are electrodes placed on the patient's skin, are configured to measure or track the operation of the heart 8, and in particular its electrical activity and are thus configured, in this example, to carry out a measurement or test, which in some aspects may be comparable to a complete or incomplete electrocardiogram, abbreviated as EKG or ECG, which is a test that measures the electrical activity of the heart. **It** is to be noted that with each heartbeat, an electrical impulse, signal or "wave" travels through the heart. This signal then causes the muscle to squeeze and pump blood from the heart. **It** is to be noted that the number of sensors in the sensor system typically varies depending on the exact implementation, but this number is typically any number from one to ten, or more specifically from three to ten or from three to eight. The recorded electrical activity signals are also configured to be sampled and digitised by the sensor system 3 or more specifically by the individual sensors 5 by using a respective data sampler and digitiser. **It** is to be noted that it is also possible to sub-sample the recorded signals later if so desired. Also, the digitising may be carried out later during the cell detection process, for example by the data processing apparatus 7.

The (digital) electrical activity signals measured or recorded by the sensors are then arranged to be fed into the data processing apparatus 7, which in the simplified example of Figure 1 comprises the following functional modules or units: a communication interface 9, a time-reversal module 10, a heart (or object or subject more broadly) modelling module (or a model generator) 11, a data analysis module 13, a memory and/or data storage module 15, and a transposing module 16. **It** is to be noted that only the functional elements useful for understanding the teachings of the present invention are shown in Figure 1. It is to be noted that the functional modules shown inside the data processing apparatus may form one or more hardware modules. The communication interface 9 is configured to communicate with the sensor system 3 and more specifically with the corresponding interface(s) of the sensor system. The time-reversal module 10 is configured to carry out a time-reversal operation for the one or more signals it receives from the sensor system 5.

Time-reversal or T-symmetry describes the symmetry of physical laws under a time-reversal transformation: *t* → *-t.* The time-reversal operation causes the original signal to flip with respect to its amplitude axis (i.e., typically the vertical axis of reference). This means that the operation results in the reflection of the signal along its amplitude axis of reference (i.e., typically the vertical axis of reference). The operation is known as the time-reversal or time reflection of the signal. In the past couple of decades, the technique has found many applications in the field of engineering, especially in source-location identification such as landmine detection and fault location in power networks.

Time-reversal operation in signal processing can be understood as a spatial focusing technique that uses the reciprocity principle. Let us imagine a signal that is sent out from a transmit location, which in the example illustrated later would be a heart cell. The signal can be picked up at several receive locations (i.e., the sensor locations). The sensors also record these received signals. Now the system can be visualised in reverse. The previous receive locations can become transmit locations. These locations (when transposed to a heart model as explained later) transmit the previously recorded signals simultaneously but in a time-reversed manner. At the target locations in the model (corresponding to the original transmitters or the heart cells in the present case), all the signals converge or focus in space at the transmit location in the model as will be explained later in more detail.

The heart modelling module 11 is arranged to form a model 17 of the object 8 to be analysed, which in the present example is the heart. The modelling module is thus arranged to generate a model of the heart such that it is in this example substantially identical in shape and/or dimensions to the heart to be analysed. The natural pacemaker of the heart, i.e., the sinoatrial node is also located in the model substantially at the same location as in the real heart. This means that the "normal" heart activity signal, which is the signal generated by the sinoatrial node, can be used to "calibrate" the computer-simulated model by comparing the localised heart cells' positions, after having fed the time-reversed signals into the model, to the actual position of the sinoatrial node in the heart to determine which one of the localised cells corresponds to the sinoatrial node.

The data analysis module 13 is arranged to implement the method of determining the cell locations transmitting electrical signals as will be explained later in more detail. In the analysis, the data analysis module 13 is configured to use the time-reversed signals obtained from the time-reversal module 10, and the model obtained from the heart modelling module 11. The memory 15 may then be used to save or store the analysis results. It may also store the results from the time-reversal module 10 and/or the heart modelling module 11. The transposing module 16 is arranged to calculate or determine the exact locations of the detected cells in the subject's body part, i.e. in the heart in this example. In other words, the transposing module 16 is arranged to transpose the detected cells to the subject's body part. The determination is advantageously carried out by using the shape and/or dimensions of the subject's body part and the generated model, as well as the location of the detected cells in the model. It is to be noted that, in this example, the various functional modules of the data processing apparatus 7 are arranged to communicate and exchange data with each other.

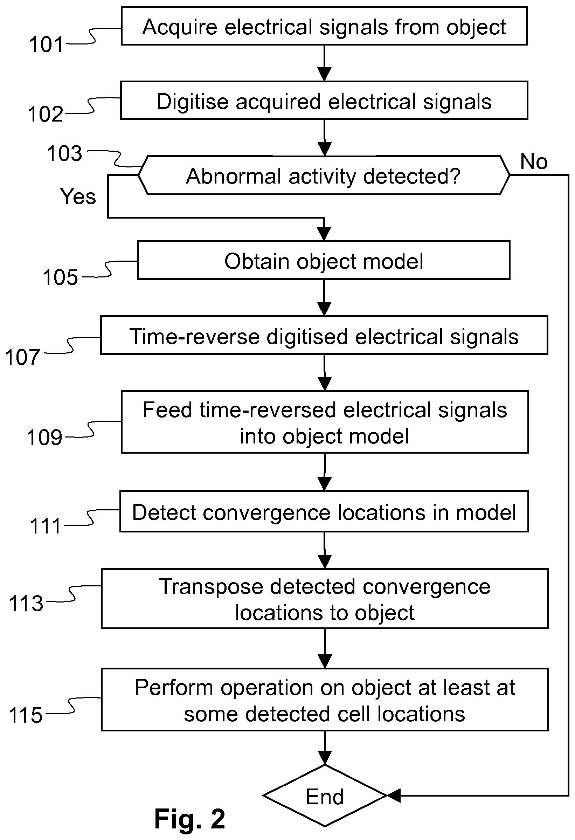

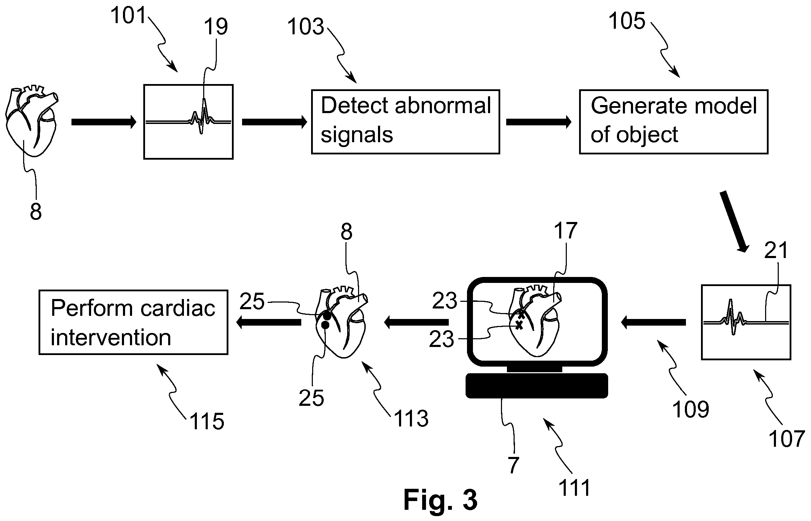

The operation of the cell detection system 1 of Figure 1 is next explained in more detail with reference to the flow charts of Figures 2 and 3. In step 101, electrical activity signals of a subject's body part, which in this specific example is a human heart, are acquired or obtained. These electrical activity signals may be pre-measured signals thus characterising pre-measured electrical activity, or, as in this non-limiting specific example, they may be recorded by the sensors 5, which are configured to measure electrical activity of the heart 8 by receiving electrical activity signals or signal waveforms from various heart cells. It is to be noted that if pre-measured electrical signals are acquired, then the cell detection system 1 does not need to include the sensor system 3 as the pre-measured electrical activity signals can be directly received by the data processing apparatus 7, and in particular by e.g. the communication interface 9.

In this example, the sensors thus record the electrical variation produced by the heart activity. It is to be noted that the conveyed or received electrical signals are a combination of the signal generated by the sinoatrial node and those of other cells. In this example, a respective sensor 5 receives electrical signals from all the signal-generating cells. The respective received electrical activity signal 19 at a respective sensor can be understood as a respective combined electrical activity signal as there are, in this example, several transmitting cells and several transmitted signals can thus be combined upon receipt at each sensor. The amplitude (which in this example is expressed in volts) of the received signal at the respective sensor against time is a signal comparable (to some extent) to the ECG usually used by cardiologists. It is to be noted that in this arrangement, at least three sensors 5 are used, which are located in different locations. However, as stated above, it would be possible to operate the system 1 so that only one or two sensors is/are used. The locations of the sensors on the patient's skin are recorded to be later used when feeding or injecting the time-reversed versions of the received signals into the simulation model 17 advantageously in corresponding locations in the model. Thus, in this example, the spatial relationship of the sensors on the patient's skin is the same or substantially the same as the spatial relationship of the injection or input locations in the model.

In step 102, the recorded electrical activity signals are sampled and digitised by the sensor system 3 or more specifically by the individual sensors 5. The sampling frequency may be chosen appropriately, and it may be e.g. between 50 Hz and 5000 Hz, or more specifically between 100 Hz and 1000 Hz. In step 103, it is determined whether or not the received electrical activity signals are abnormal. This can be carried out for instance by comparing the received electrical activity signals with some normal signals which represent the heart activity of a healthy heart. These reference signals may be stored in the data storage module 15. If the received electrical activity signals are determined to be normal, then the process can be terminated. If on the other hand, it is determined that the signals show some anomalies, then the process progresses to step 105. However, it is to be noted that step 103 is optional.

In step 105, a simulation model 17 corresponding to the patient's heart is generated by the heart modelling module 11. This step thus also comprises calibrating the model with the shape and/or dimensions of the heart and/or parameters relating to the sinoatrial node of the heart (such as the position and/or functioning of the sinoatrial node). The model 17 may be considered to be a passive element, which is responsive to its input signals as explained later. This step also comprises feeding the received electrical activity signals 19 into the data processing apparatus 7, if the data processing apparatus 7 is separate from the sensor system 3.

In step 107, the time-reversal module 10 time-reverses the received electrical activity signals 19 to obtain time-reversed electrical activity signals 21. This means that the recorded signals are processed by a computer, which in this example is the data processing apparatus 7. When the signals are time-reversed, this means that for each set of data, the order of the reception of each data element (once any analogue signals have been sampled and digitised) is reversed, i.e., the last data element becomes the first, etc. When carrying out the time-reversal of the signals, a first received electrical activity signal from a first sensor is time-reversed to obtain a first time-reversed electrical activity signal, a second received electrical activity signal from a second sensor is time-reversed to obtain a second time-reversed electrical activity signal, etc.

In step 109, the time-reversed electrical activity signals 21 are fed or input into the generated model 17. More specifically, the first time-reversed electrical activity signal is fed into the model at a first model location, while the second time-reversed electrical activity signal is fed into the model at a second model location, etc. The location of each sensor on the patient's skin is used to calculate the corresponding input location or point on the envelope (i.e., on the outer layer or surface) of the simulated model of the heart. The time-reversed signals 21 are thus input into the model as electrical signals on its envelope. Advantageously all the input locations are different from each other. As the signals are already time-reversed, they are in this example fed into the model 17 simultaneously.

In step 111, the simulation model is run with the input signals described above. As a result of the injection, the injected signals then spatially focus or converge in the model at the locations 23 which correspond to the heart cell locations where electrical signals are generated. In this example, one of these locations 23 corresponds to the sinoatrial node, and as its location is already known, the remaining convergence locations 23 can thus be used to indicate the locations of the faulty or abnormal cells. The generated electrical waves thus focus on the original cell or cells responsible for the initially transmitted electrical signals. The locations of these cells in the simulated model are identified and/or recorded. These locations can then be used by a cardiologist treating the "real" heart.

By knowing the spatial correspondence between the real heart and the model, in step 113, the convergence locations can then be transposed to the real heart 8 to determine the exact locations of the faulty or abnormal cells 25 in the heart. All the locations of the cells generating electrical activity can in this manner be determined in step 113. In other words, by time-reversing the electrical activity signals received by the sensors 5, and which are recorded during abnormal beatings (arrhythmic phases), the faulty cell(s) can be located by identifying the focusing points of the injected time-reversed electrical activity signals 21. The location of these cells on the model is then transposed to the patient's heart by using the transposing module 16 in order to optionally burn them in a subsequent step, i.e. in step 115. Thus, after having detected the cell locations, a cardiac intervention may be initiated during which an ablation catheter inside the heart may be guided to the detected cell locations to carry out cardiac ablation.

It is to be noted that some of the steps illustrated in the flow charts of Figures 2 and 3 are optional, in particular steps 103, 113 and 115. Furthermore, the order of the steps may be different from the order illustrated in the flow charts of Figures 2 and 3. For instance, step 105 may be carried out at any moment before step 109, and step 102 could be carried out after step 103. The above process may also be altered by modifying one or more of the individual steps. For example, it is possible to filter the received electrical signals to improve the signal quality and/or so that the aim of the filtering would be to exclude or minimise the contribution of the sinoatrial node (or any other signal source). This would mean that the filtered signals would not include the signal generated by the sinoatrial node (or any other signal that is to be filtered out). The filtering could instead (or in addition) be carried out on the time-reversed electrical activity signals. Depending on at which stage the filtering is carried out, it may be performed by the sensor system 3 and/or the data processing apparatus 7 by using a signal filtering arrangement. To be able to successfully filter the signals, some property of the signal source to be excluded is advantageously used for this purpose. This property could e.g. be the signal frequency of the signal generated by the signal source to be filtered out. The waveform of a signal portion visible on the received electrical activity signal could also or in addition be used when carrying out the filtering.

It is to be noted that there are many other possible applications for the teachings of the present invention. For instance, the method described in this invention can be transposed to other organs of the human or animal body with circulation of electrical signals, such as muscular fibres, brain or nervous system. The localisation of sources of "unusual patterns" can be achieved by time-reversing the collected electrical signals and using the time-reversed signals in a simulated model of the organ.

The above described method may be carried out by suitable circuits or circuitry. The terms "circuits" and "circuitry" refer to physical electronic components or modules (e.g. hardware), and any software and/or firmware ("code") that may configure the hardware, be executed by the hardware, and or otherwise be associated with the hardware. The circuits may thus be configured or operable to carry out or they comprise means for carrying out the required method as described above.

While the invention has been illustrated and described in detail in the drawings and foregoing description, such illustration and description are to be considered illustrative or exemplary and not restrictive, the invention being not limited to the disclosed embodiment. example, all or part of the computing (i.e., the data processing) according to the teachings of the present invention could be implemented as cloud computing by using computing power over the Internet.

In the claims, the word "comprising" does not exclude other elements or steps, and the indefinite article "a" or "an" does not exclude a plurality. Any reference signs in the claims should not be construed as limiting the scope of the invention.

## Claims

1. A data processing apparatus (7) for locating one or more cells (25) generating an electrical anomaly in a subject's body part (8), the apparatus comprising means for:
• obtaining one or more electrical signals (19) resulting from electrical activity of the one or more cells (25) in the subject's body part (8);
• time-reversing the one or more electrical signals (19) for obtaining one or more time-reversed electrical signals (21);
• feeding the one or more time-reversed electrical signals (21) into a simulation model (17) of the subject's body part, the simulation model (17) being configured to model anatomy and electrical activity of the subject's body part (8), to cause the time-reversed electrical signals (21) when fed into the simulation model (17) to converge in the simulation model at one or more convergence locations (23); and
• detecting the one or more convergence locations (23) in the simulation model (17) thereby allowing locating the one or more cells (25) generating the electrical anomaly in the subject's body part.

2. The data processing apparatus (7) according to claim 1, wherein the data processing apparatus (7) further comprises means for transposing (16) the convergence locations (23) to the subject's body part (8).

3. The data processing apparatus (7) according to claim 1 or 2, wherein the data processing apparatus (7) comprises a computer model generator for generating the simulation model (17).

4. The data processing apparatus (7) according to claim 3, wherein the simulation model (17) is configured to be calibrated with the subject's body part (8) by considering dimensions of the subject's body part and/or one or more physiologically normal electrical signal(s) generated by the subject's body part (8).

5. The data processing apparatus (7) according to any one of the preceding claims, wherein the number of electrical signals is at least three.

6. The data processing apparatus (7) according to any one of the preceding claims, wherein the subject's body part (8) is a heart, and the electrical anomaly is a cardiac electrical anomaly, and wherein the cardiac electrical anomaly is cardiac arrhythmia.

7. The data processing apparatus (7) according to any one of the preceding claims, wherein the time-reversed electrical signals (21) are configured to be fed into the simulation model (17) substantially simultaneously.

8. The data processing apparatus (7) according to any one of the preceding claims, wherein the time-reversed electrical signals (21) are configured to be fed into the simulation model (17) at different locations.

9. A system (1) for locating one or more cells (25) generating an electrical anomaly in a subject's body part (8), the system (1) comprising the data processing apparatus (7) according to any one of the preceding claims, and a sensor system (3) comprising a set of sensors (5) for recording the one or more electrical signals (19) resulting from electrical activity of the subject's body part.

10. The system (1) according to claim 9, wherein the sensor system (3) is arranged to send the recorded one or more electrical signals (19) to the data processing apparatus over a wireless communication link between the sensor system (5) and the data processing apparatus (7).

11. The system (1) according to claim 9 or 10, wherein the set of sensors comprises one or more sensors (5) each located at a respective sensing location such that the one or more sensing locations have a first spatial relationship with respect to each other, wherein the one or more time-reversed electrical signals (21) are arranged to be fed into the simulation model (17) at a respective feed location such that the one or more feed locations have a second spatial relationship with respect to each other, and wherein the first spatial relationship is substantially identical to the second spatial relationship.

12. The system (1) according to any one of claims 9 to 11, wherein the system (1) further comprises a signal filter for filtering the one or more electrical signals (19) and/or the one or more time-reversed electrical signals (21) to improve signal quality and/or filter out the effect of the one or more cells (25) on the respective signal.

13. The system (1) according to any one of claims 9 to 12, wherein the system (1) further comprises a data digitiser for sampling and digitising the one or more electrical signals (19).

14. A computer-implemented method for locating one or more cells (25) generating an electrical anomaly in a subject's body part (8), the method comprising the steps of:
• obtaining (101) one or more pre-measured electrical signals (19) resulting from electrical activity of the one or more cells (25) in the subject's body part (8);
• time-reversing (107) the one or more electrical signals (19), thereby obtaining one or more time-reversed electrical signals (21);
• feeding (109) the one or more time-reversed electrical signals (21) into a simulation model (17) of the subject's body part, the simulation model (17) being configured to model anatomy and electrical activity of the subject's body part (8), to cause the time-reversed electrical signals (21) when fed into the simulation model (17) to converge in the simulation model (17) at one or more convergence locations (23); and
• detecting (111) the one or more convergence locations (23) in the simulation model (17) thereby allowing locating the one or more cells (23) generating the electrical anomaly in the subject's body part.

15. A non-transitory computer program product comprising instructions for implementing the steps of the method according to claim 14 when loaded and run on computing means of a computing device (7).

## Patentansprüche

1. Eine Datenverarbeitungsvorrichtung (7) zum Lokalisieren einer oder mehrerer Zellen (25), die eine elektrische Anomalie in einem Körperteil (8) eines Subjekts erzeugen, wobei die Vorrichtung Mittel umfasst zum:
• Erfassen eines oder mehrerer elektrischer Signale (19), die aus der elektrischen Aktivität der einen oder mehrerer Zellen (25) im Körperteil (8) des Subjekts resultieren;
• Zeitumkehrung des oder der elektrischen Signale (19), um ein oder mehrere zeitumgekehrte elektrische Signale (21) zu erhalten;
• Einspeisen des oder der zeitumgekehrten elektrischen Signale (21) in ein Simulationsmodell (17) des Körperteils des Subjekts, wobei das Simulationsmodell (17) so konfiguriert ist, dass es die Anatomie und elektrische Aktivität des Körperteils (8) des Subjekts modelliert, um zu bewirken, dass die zeitumgekehrten elektrischen Signale (21) im Simulationsmodell (17) an einer oder mehreren Konvergenzstellen (23) konvergieren; und
• Erkennen der einen oder mehrerer Konvergenzstellen (23) im Simulationsmodell (17), wodurch das Lokalisieren der einen oder mehrerer Zellen (25), die die elektrische Anomalie im Körperteil (8) des Subjekts erzeugen, ermöglicht wird.

2. Die Datenverarbeitungsvorrichtung (7) nach Anspruch 1, wobei die Datenverarbeitungsvorrichtung (7) ferner Mittel zum Übertragen (16) der Konvergenzstellen (23) auf das Körperteil (8) des Subjekts umfasst.

3. Die Datenverarbeitungsvorrichtung (7) nach Anspruch 1 oder 2, wobei die Datenverarbeitungsvorrichtung (7) einen Computermodellgenerator zum Erzeugen des Simulationsmodells (17) umfasst.

4. Die Datenverarbeitungsvorrichtung (7) nach Anspruch 3, wobei das Simulationsmodell (17) so konfiguriert ist, dass es anhand der Abmessungen des Körperteils (8) des Subjekts und/oder eines oder mehrerer physiologisch normaler elektrischer Signale, die vom Körperteil (8) erzeugt werden, kalibriert wird.

5. Die Datenverarbeitungsvorrichtung (7) nach einem der vorhergehenden Ansprüche, wobei die Anzahl der elektrischen Signale mindestens drei beträgt.

6. Die Datenverarbeitungsvorrichtung (7) nach einem der vorhergehenden Ansprüche, wobei das Körperteil (8) des Subjekts ein Herz ist, die elektrische Anomalie eine kardiale elektrische Anomalie ist und die kardiale Anomalie eine Herzrhythmusstörung ist.

7. Die Datenverarbeitungsvorrichtung (7) nach einem der vorhergehenden Ansprüche, wobei die zeitumgekehrten elektrischen Signale (21) im Wesentlichen gleichzeitig in das Simulationsmodell (17) eingespeist werden.

8. Die Datenverarbeitungsvorrichtung (7) nach einem der vorhergehenden Ansprüche, wobei die zeitumgekehrten elektrischen Signale (21) an verschiedenen Stellen in das Simulationsmodell (17) eingespeist werden.

9. Ein System (1) zum Lokalisieren einer oder mehrerer Zellen (25), die eine elektrische Anomalie in einem Körperteil (8) eines Subjekts erzeugen, wobei das System (1) die Datenverarbeitungsvorrichtung (7) nach einem der vorhergehenden Ansprüche und ein Sensorsystem (3) umfasst, das einen Satz von Sensoren (5) zum Aufzeichnen des oder der elektrischen Signale (19) enthält, die aus der elektrischen Aktivität des Körperteils (8) resultieren.

10. Das System (1) nach Anspruch 9, wobei das Sensorsystem (3) so angeordnet ist, dass es die aufgezeichneten elektrischen Signale (19) über eine drahtlose Kommunikationsverbindung zwischen dem Sensorsystem (5) und der Datenverarbeitungsvorrichtung (7) an die Datenverarbeitungsvorrichtung sendet.

11. Das System (1) nach Anspruch 9 oder 10, wobei der Satz von Sensoren einen oder mehrere Sensoren (5) umfasst, die jeweils an einer jeweiligen Erfassungsstelle angeordnet sind, sodass die Erfassungsstellen eine erste räumliche Beziehung zueinander aufweisen, wobei die zeitumgekehrten elektrischen Signale (21) an jeweiligen Einspeisepunkten in das Simulationsmodell (17) eingespeist werden, sodass die Einspeisepunkte eine zweite räumliche Beziehung zueinander aufweisen, wobei die erste räumliche Beziehung im Wesentlichen identisch mit der zweiten räumlichen Beziehung ist.

12. Das System (1) nach einem der Ansprüche 9 bis 11, wobei das System (1) ferner einen Signalfilter zum Filtern des oder der elektrischen Signale (19) und/oder des oder der zeitumgekehrten elektrischen Signale (21) umfasst, um die Signalqualität zu verbessern und/oder die Wirkung der einen oder mehrerer Zellen (25) auf das jeweilige Signal herauszufiltern.

13. Das System (1) nach einem der Ansprüche 9 bis 12, wobei das System (1) ferner einen Datendigitalisierer zum Abtasten und Digitalisieren des oder der elektrischen Signale (19) umfasst.

14. Ein computerimplementiertes Verfahren zum Lokalisieren einer oder mehrerer Zellen (25), die eine elektrische Anomalie in einem Körperteil (8) eines Subjekts erzeugen, wobei das Verfahren folgende Schritte umfasst:
• Erfassen (101) eines oder mehrerer vorab gemessener elektrischer Signale (19), die aus der elektrischen Aktivität der Zellen (25) im Körperteil (8) resultieren;
• Zeitumkehrung (107) des oder der elektrischen Signale (19), wodurch ein oder mehrere zeitumgekehrte elektrische Signale (21) erhalten werden;
• Einspeisen (109) des oder der zeitumgekehrten elektrischen Signale (21) in ein Simulationsmodell (17) des Körperteils (8) des Subjekts, wobei das Simulationsmodell (17) so konfiguriert ist, dass es die Anatomie und elektrische Aktivität des Körperteils modelliert, um zu bewirken, dass die zeitumgekehrten elektrischen Signale (21) im Simulationsmodell (17) an einer oder mehreren Konvergenzstellen (23) konvergieren; und
• Erkennen (111) der einen oder mehrerer Konvergenzstellen (23) im Simulationsmodell (17), wodurch das Lokalisieren der einen oder mehrerer Zellen (25), die die elektrische Anomalie erzeugen, ermöglicht wird.

15. Ein nicht-flüchtiges Computerprogrammprodukt, das Anweisungen umfasst, um die Schritte des Verfahrens nach Anspruch 14 auszuführen, wenn es auf den Rechenmitteln eines Computergeräts (7) geladen und ausgeführt wird.

## Revendications

1. Un appareil de traitement de données (7) pour localiser une ou plusieurs cellules (25) générant une anomalie électrique dans une partie du corps (8) d'un individu, l'appareil comprenant des moyens pour :
• obtenir un ou plusieurs signaux électriques (19) résultant de l'activité électrique de la ou des cellules (25) dans la partie du corps (8) de l'individu ;
• inverser temporellement le ou les signaux électriques (19) afin d'obtenir un ou plusieurs signaux électriques inversés dans le temps (21) ;
• introduire le ou les signaux électriques inversés dans le temps (21) dans un modèle de simulation (17) de la partie du corps de l'individu, le modèle de simulation (17) étant configuré pour modéliser l'anatomie et l'activité électrique de la partie du corps (8) de l'individu, de manière à provoquer la convergence, dans le modèle de simulation, des signaux électriques inversés dans le temps (21) en un ou plusieurs emplacements de convergence (23) ; et
• détecter le ou les emplacements de convergence (23) dans le modèle de simulation (17), permettant ainsi de localiser la ou les cellules (25) générant l'anomalie électrique dans la partie du corps (8) de l'individu.

2. L'appareil de traitement de données (7) selon la revendication 1, dans lequel l'appareil de traitement de données (7) comprend en outre des moyens pour transposer (16) les emplacements de convergence (23) sur la partie du corps (8) de l'individu.

3. L'appareil de traitement de données (7) selon la revendication 1 ou 2, dans lequel l'appareil de traitement de données (7) comprend un générateur de modèle informatique destiné à générer le modèle de simulation (17).

4. L'appareil de traitement de données (7) selon la revendication 3, dans lequel le modèle de simulation (17) est configuré pour être calibré avec la partie du corps (8) de l'individu en tenant compte des dimensions de la partie du corps et/ou d'un ou plusieurs signaux électriques physiologiquement normaux générés par la partie du corps (8).

5. L'appareil de traitement de données (7) selon l'une quelconque des revendications précédentes, dans lequel le nombre de signaux électriques est au moins égal à trois.

6. L'appareil de traitement de données (7) selon l'une quelconque des revendications précédentes, dans lequel la partie du corps (8) de l'individu est un cœur, l'anomalie électrique est une anomalie cardiaque, et l'anomalie cardiaque est une arythmie.

7. L'appareil de traitement de données (7) selon l'une quelconque des revendications précédentes, dans lequel les signaux électriques inversés dans le temps (21) sont destinés à être introduits dans le modèle de simulation (17) de manière sensiblement simultanée.

8. L'appareil de traitement de données (7) selon l'une quelconque des revendications précédentes, dans lequel les signaux électriques inversés dans le temps (21) sont destinés à être introduits dans le modèle de simulation (17) en différents emplacements.

9. Un système (1) pour localiser une ou plusieurs cellules (25) générant une anomalie électrique dans une partie du corps (8) d'un individu, le système (1) comprenant l'appareil de traitement de données (7) selon l'une quelconque des revendications précédentes, et un système de capteurs (3) comprenant un ensemble de capteurs (5) pour enregistrer le ou les signaux électriques (19) résultant de l'activité électrique de la partie du corps (8) de l'individu.

10. Le système (1) selon la revendication 9, dans lequel le système de capteurs (3) est configuré pour transmettre le ou les signaux électriques enregistrés (19) à l'appareil de traitement de données (7) via une liaison de communication sans fil entre le système de capteurs (5) et l'appareil de traitement de données (7).

11. Le système (1) selon la revendication 9 ou 10, dans lequel l'ensemble de capteurs comprend un ou plusieurs capteurs (5) chacun disposé à un emplacement de détection respectif, de sorte que le ou les emplacements de détection présentent une première relation spatiale entre eux, dans lequel le ou les signaux électriques inversés dans le temps (21) sont introduits dans le modèle de simulation (17) à un emplacement d'introduction respectif, de sorte que le ou les emplacements d'introduction présentent une seconde relation spatiale entre eux, et dans lequel la première relation spatiale est sensiblement identique à la seconde relation spatiale.

12. Le système (1) selon l'une quelconque des revendications 9 à 11, dans lequel le système (1) comprend en outre un filtre de signaux destiné à filtrer le ou les signaux électriques (19) et/ou le ou les signaux électriques inversés dans le temps (21) afin d'améliorer la qualité du signal et/ou d'éliminer l'effet de la ou des cellules (25) sur le signal respectif.

13. Le système (1) selon l'une quelconque des revendications 9 à 12, dans lequel le système (1) comprend en outre un numériseur de données destiné à échantillonner et numériser le ou les signaux électriques (19).

14. Un procédé mis en oeuvre par ordinateur pour localiser une ou plusieurs cellules (25) générant une anomalie électrique dans une partie du corps (8) d'un individu, le procédé comprenant les étapes consistant à :
• obtenir (101) un ou plusieurs signaux électriques préalablement mesurés (19) résultant de l'activité électrique de la ou des cellules (25) dans la partie du corps (8) de l'individu ;
• inverser temporellement (107) le ou les signaux électriques (19), obtenant ainsi un ou plusieurs signaux électriques inversés dans le temps (21) ;
• introduire (109) le ou les signaux électriques inversés dans le temps (21) dans un modèle de simulation (17) de la partie du corps (8) de l'individu, le modèle de simulation (17) étant configuré pour modéliser l'anatomie et l'activité électrique de la partie du corps (8) de l'individu, de manière à provoquer la convergence, dans le modèle de simulation (17), des signaux électriques inversés dans le temps (21) en un ou plusieurs emplacements de convergence (23) ; et
• détecter (111) le ou les emplacements de convergence (23) dans le modèle de simulation (17), permettant ainsi de localiser la ou les cellules (25) générant l'anomalie électrique dans la partie du corps (8) de l'individu.

15. Un produit programme d'ordinateur non transitoire comprenant des instructions pour exécuter les étapes du procédé selon la revendication 14 lorsqu'il est chargé et exécuté sur des moyens de calcul d'un dispositif informatique (7).
